# EUROPEAN PATENT APPLICATION

(11) **EP 2 417 976 A1**
(43) Date of publication of application: **15.02.2012**
(21) Application number: 10761535.3
(22) Date of filing: 08.03.2010
(51) Int. Cl.: A61K 31/7048, A23L 1/30, A61K 31/353, A61K 36/18, A61P 25/20, C07D 407/04, C07H 17/04

(54) **PROCESS FOR PRODUCTION OF ORALLY INGESTIBLE COMPOSITION CONTAINING ARABINOSYL VITEXIN, AND USE OF THE COMPOSITION**

(30) Priority: 10.04.2009 JP 2009095445
(71) Applicant: Fuji-Sangyo Co., Ltd., Marugame-shi, Kagawa 763-0071 (JP)
(72) Inventor: KUSANO, Shuichi, Sakaide-shi Kagawa 762-0024 (JP)
(74) Representative: Gille Hrabal
(86) International application number: PCT/JP2010/053752
(87) International publication number: WO 2010/116834

(57) **Abstract**

An object of the present invention is to find a plant that contains a C-glucoside flavonoid having an excellent physiological activity at a high content among plants which are readily available and highly safe from the viewpoint of oral ingestion, and to produce an orally ingestible composition (in particular, a food, a beverage, or a pharmaceutical preparation) containing the C-glucoside flavonoid as an active ingredient. Provided are : aproductionmethod for an orally ingestible composition containing an arabinofuranosyl vitexin and/or a hydrolysate thereof, i.e., vitexin, and having a sedative activity and/or a sleep-inducing activity, the method including using *Basella alba* as a raw material; a food or beverage, including an orally ingestible composition obtained by the production method; and a drug, including a composition obtained by the production method and having a sedative activity and/or a sleep-inducing activity.

## Description

### Technical Field

The present invention relates to a production method for an orally ingestible composition containing an arabinofuranosyl vitexin and/or a hydrolysate thereof, i.e., vitexin, the method including using *Basella alba* as a rawmaterial, and more specifically, to a production method for an orally ingestible composition containing the compound as an active ingredient and having a sedative activity and/or a sleep-inducing activity.
The present invention further relates to a food, a beverage, or a pharmaceutical preparation formed of the orally ingestible composition.

### Background Art

Flavonoid is a collective term for compounds which are biosynthesized mainly in plants and each of which has a flavan-derivative skeleton. Some species of flavonoids have excellent physiological activities.
In most cases, a flavonoid derived from a plant is generally present in a form of 'O-glucoside flavonoid' (a flavonoid glycoside having an O-glucoside skeleton), which is easily affected by an enzymatic activity, or 'aglycone' (a flavonoid containing no constituent sugar), which is insoluble in water.

On the other hand, a "C-glucoside flavonoid" (a flavonoid glycoside having a C-glucoside skeleton) is linked to a constituent sugar via a C-glycoside bond, and hence the compound is difficult to be decomposed by heat, hydrolysis, an enzyme, or the like and is highly stable. Moreover, the compound is a glycoside and hence has excellent water solubility. In addition, some species of C-glucoside flavonoids have recently been known to have excellent physiological activities for antioxidation, cell protection, anti-inflammation, anti-cancer, sleeping, anti-anxiety, and the like (see Non Patent Literature 1).
However, there are quite a few plants used as a raw material that contain C-glucoside flavonoid, and further, the content of the compound in such plants is very small (Non Patent Literature 2), and the development of an efficient extraction method has been attempted (Patent Literature 1) but the method is unsatisfactory. For example, a leaf of a plant called *Cotoneaster thymaefolia* (family Rosaceae, genus Cotoneaster, a shrub found in China and Himalaya) contains 2"-O-α-D-arabinofuranosyl vitexin, which is one kind of C-glucoside flavonoid (see Non Patent Literature 3), but it is difficult to obtain a large amount of the plant used as the raw material. In addition, the plant is not an edible plant, and it is not realistic to use the plant as the raw material for extraction of the C-glucoside flavonoid.

Therefore, in most cases, the flavonoid is used as follows. The flavonoid is obtained by extraction or purification from a plant containing the flavonoid in the form of O-glucoside flavonoid, which is easily decomposed, or in the form of aglycone, which is insoluble in water, and is used in a functional material or the like. Thus, an effective use of the C-glucoside flavonoid has not been developed.

### Citation List

### Patent Literature

[Patent Literature 1] JP 2006-265250 A

### Non Patent Literature

[Non Patent Literature 1] Cell Biol Int . (2009), 33(2), 247-52
[Non Patent Literature 2] Journal of the Japanese Society for Food Science and Technology (2003), 50(1), 32-34
[Non Patent Literature 3] Phytochemistry (1994), 35(5), 1381-2

### Summary of Invention

### Technical Problem

An object of the present invention is to solve the above-mentioned problems, to find a plant containing a C-glucoside flavonoid 'having an excellent physiological activity' at a high content among plants which are 'readily available' and 'highly safe from the viewpoint of oral ingestion,' and to produce an orally ingestible composition (in particular, a food, a beverage, or a pharmaceutical preparation) containing the C-glucoside flavonoid as an active ingredient.

### Solution to Problem

In view of such circumstances, the inventor of the present invention has searched a variety of plants having flavonoids, and as a result, has found that *Basella alba,* which is an edible plant as a leaf vegetable, contains the C-glucoside flavonoid, i.e., an arabinofuranosyl vitexin at a high content. The inventor has also found that the compound has a structure of 2 "-O-α-D-arabinofuranosyl vitexin.

When thiopental as a barbital-based anesthetic is administered to a mouse at a dose lower than a dose for loss of righting reflex, the mouse moves violently. However, the inventor of the present invention has found that preadministration of the arabinofuranosyl vitexin can suppress such behavior and that the arabinofuranosyl vitexin has sedative and sleep-inducing activities as physiological activities.
The inventor has further found that the arabinofuranosyl vitexin can be easily separated into an arabinose moiety and vitexin (a main body of C-glucoside flavonoid) by hydrolysis.

The inventor of the present invention has firstly found the above-mentioned findings. In addition, the inventor has completed the present invention based on the findings.
That is, a first aspect of the present invention provides a production method for an orally ingestible composition containing an arabinofuranosyl vitexin and/or a hydrolysate thereof, i.e., vitexin, the method comprising using *Basella alba* as a raw material.
A second aspect of the present invention provides a production method for an orally ingestible composition according to the first aspect, in which the arabinofuranosyl vitexin is 2"-O-α-D-arabinofuranosyl vitexin.
A third aspect of the present invention provides a production method for an orally ingestible composition according to the first or second aspect, in which the production method for an orally ingestible composition comprises the steps of: treating *Basella alba* used as the raw material by any one of cutting, fragmentating, and grinding; and performing solution extraction to prepare an extract.
A fourth aspect of the present invention provides a production method for an orally ingestible composition according to any one of the first to third aspects, in which the production method for an orally ingestible composition comprises the step of performing solution extraction with ethanol or ethanol having a final concentration of 55% or more to prepare an extract.
A fifth aspect of the present invention provides a production method for an orally ingestible composition according to any one of the first to fourth aspect, in which the production method for an orally ingestible composition includes the step of purifying the resultant extract using a column after obtaining the extract.
A sixth aspect of the present invention provides a production method for an orally ingestible composition according to any one of the first to fifth aspect, in which the orally ingestible composition contains 2"-O-α-D-arabinofuranosyl vitexin and/or a hydrolysate thereof, i.e., vitexin as an active ingredient and has a sedative activity and/or a sleep-inducing activity.
A seventh aspect of the present invention provides a food or beverage, comprising an orally ingestible composition obtained by the production method according to any one of the first to sixth aspects.
An eighth aspect of the present invention provides a drug, comprising an orally ingestible composition obtained by the production method according to the sixth aspect.
A ninth aspect of the present invention provides a drug, containing 2"-O-α-D-arabinofuranosyl vitexin as an active ingredient and having a sedative activity and/or a sleep-inducing activity.

### Advantageous Effects of Invention

The present invention enables the production of an orally ingestible composition having 'sedative and sleep-inducing activities (excellent physiological activities) 'using a plant which is 'readily available' and 'highly safe from the viewpoint of oral ingestion' as a raw material.
That is, in the present invention, *Basella alba,* which is readily available as a leaf vegetable, is used as a raw material for extraction of an active ingredient, and hence the raw material is very readily available. Moreover, the raw material is highly safe from the viewpoint of oral ingestion.
Further, the compound which is the active ingredient of the present invention (C-glucoside flavonoid) has properties of 'being highly soluble in water' and 'being difficult to be decomposed and highly stable as a glycoside,' and hence the compound can be applied to a wide range of processed products produced by a variety of treatments and the like.

The present invention further enables the production of a food or beverage including the orally ingestible composition. The present invention also enables the production of a pharmaceutical preparation including the orally ingestible composition.

### Brief Description of Drawings

[FIG. 1] FIG. 1 is a view showing the structure of 2"-O-α-D-arabinofuranosyl vitexin determined in Example 1.
[FIG. 2] FIG. 2 is a graph showing the sedative and sleep-inducing activity of 2"-O-α-D-arabinofuranosyl vitexin in Example 2.

### Description of Embodiments

Hereinafter, the present invention is described in detail.
The present invention relates to a production method for an orally ingestible composition containing an arabinofuranosyl vitexin and/or a hydrolysate thereof, i.e., vitexin, the method including using *Basella alba* as a raw material, and specifically, to a production method for an orally ingestible composition containing the above-mentioned compound as an active ingredient and having a sedative activity and/or a sleep-inducing activity.

The raw material which may be used in the present invention is *Basella alba* belonging to the family *Basellaceae,* the genus *Basella.* Further, all plants belonging to the species *Basella alba* (for example, blue-stem species and red-stem species) may be used regardless of the breed and strain.
Any part of the plant body of *Basella alba* may be used as the raw material of the present invention, but aerial parts (mainly leaf and stem) are preferred from the viewpoint of the content. It should be noted that the aerial parts (mainly leaf and stem) are edible parts of *Basella alba* as a leaf vegetable.
Moreover, a raw plant body, a dried product, a heated product, a frozen product, and the like may be used, and in particular, a raw plant body and a dried product are preferably used. It should be noted that in the case where a 'solvent containing no water' (such as 100% ethanol) is used in the below-mentioned extraction step, the dried product containing no water is desirably used as the raw material.

In the production method of the present invention, *Basella alba* used as the raw material may be used as it is, but is preferably subjected to a treatment by any one of cutting, fragmentating, and grinding.
This step includes a variety of treatments for *Basella alba* used as the raw material, such as cutting into large pieces, cutting into small pieces, crushing, and grinding. This step is preferably carried out by cutting the raw material into large pieces with sizes of about one to a few centimeters.

The aerial parts of *Basella alba* (raw material) contain the arabinofuranosyl vitexin (in particular, 2"-O-α-D-arabinofuranosyl vitexin) at a content of about 0.5 to 1.5 mass% (specifically about 1 mass%) based on dry weight, which is very high for a natural product.
Therefore, the arabinofuranosyl vitexin may be contained in production of the orally ingestible composition of the present invention by simply blending the plant body of *Basella alba* used as the raw material (preferably, a product obtained by treating the raw material by any one of cutting, fragmentating, and grinding as described above), and for example, the raw material (preferably, a product obtained by treating the raw material by any one of cutting, fragmentating, and grinding as described above) may be added as it is to produce a composition.
However, from the viewpoint of the purity, it is desirable to obtain an extract by solution extraction in the production of the orally ingestible composition of the present invention.

The raw material may be directly subjected to the solution extraction step. It is more preferable, from the viewpoint of effective extraction, that the raw material is treated by any one of cutting, fragmentating, and grinding prior to solution extraction.
The solvent to be used in the solution extraction step may be water, a buffer, an organic solvent, or a water-containing solvent thereof. Examples of the organic solvent include: a lower aliphatic alcohol such as ethanol, methanol, isopropanol, or butanol; acetone; ethyl acetate; and chloroform.
Of those solvents, water, ethanol, orwater-containing ethanol is particularly preferred from the viewpoints of extraction efficiency, handling, and safety.
Further, it is particularly preferable to carry out the extraction using ethanol having a final concentration of 55% or more, preferably 60% or more, more preferably 80% or more because it is possible to suppress elution of impurities, i.e., polysaccharides and to improve the extraction efficiency of the arabinofuranosyl vitexin.

With regard to extraction conditions, the extraction may be carried out by adding the solvent in an amount 1 to 50 times (weight ratio), preferably 2 to 15 times that of the raw material (preferably a fragmented product) and subjecting the mixture to immersion or shaking for 5 minutes to one month, preferably 20 minutes to one week under a temperature condition ranging from 0°C to the boiling point of the solvent, preferably from room temperature to a temperature equal to or lower than the boiling point of the solvent.
The resultant extract may be freeze-dried or dried using an evaporator or the like to prepare a concentrated and dried product.
Further, the solution extraction step may be carried out in a plurality of times using a plurality of different solvents. In particular, in the case where the first extraction is carried out using water or a low concentration of a water-containing alcohol, the extraction efficiency of 2"-O-α-D-arabinofuranosyl vitexin can be improved by subsequently performing extraction using ethanol having a concentration equal to or higher than the above-mentioned specific concentration.

The extraction may involve a reaction of hydrolyzing the 'arabinofuranosyl vitexin' extracted from the above-mentioned raw material by using a solvent (such as water or a buffer) containing an enzyme or an acid (such as hydrochloric acid) to thereby separate 'vitexin' which is a main body of the C-glucoside flavonoid.
It shouldbe noted that the hydrolysis treatment with the enzyme or acid may be carried out as an independent step after the extraction step and may be carried out after the below-mentioned purification step.

The extract obtained as described above or a concentrated and dried product obtained by drying the extract may be used without any further treatment as the orally ingestible composition of the present invention.
Alternatively, the extract or product may be subjected to the purification step to improve the purity.

The purification step can improve the purity by liquid-liquid separation extraction or by column purification using, for example, silica gel, chemically modified silica gel, activated carbon, or a synthetic adsorption resin carrier.
As an example, purification conditions for improving the purity of "2"-O-α-D-arabinofuranosyl vitexin" are shown.
First, water-soluble matter of the 'concentrated and dried product' of the extract not subjected to the hydrolysis treatment (specifically, an extract obtained by extraction with ethanol having a final concentration of 60% or more) is charged to a column including a porous synthetic adsorption resin (specifically, DIAION HP-20 [manufactured by Mitsubishi Chemical Corporation]) equilibrated with water. Then, components eluted with water are removed, and then a liquid eluted with 15 to 30% ethanol (specifically, about 20% ethanol) is collected, to thereby obtain a "2"-O-α-D-arabinofuranosyl vitexin-containing composition (purified product)" having an improved purity.
It should be noted that, when the extraction and purification are carried out under the preferred conditions described in the parentheses in the foregoing, a 2"-O-α-D-arabinofuranosyl vitexin-containing composition (purified product) having a purity of 80% or more can be obtained.
Moreover, an "arabinofuranosyl vitexin having another molecular structure" and a "hydrolysate thereof, i.e., vitexin" may be purified in almost the same manner as described above except that the preferred range of ethanol is slightly different from the above-mentioned one.

Further, the arabinofuranosyl vitexin-containing composition (purified product) or vitexin-containing composition (purified product) obtained as described above may further be subjected to thin-layer chromatography (TLC) [chloroform/methanol/water (65:25:4)] or ODS-HPLC using a mixed solvent of 20% (v/v) acetonitrile in water, and target peaks are collected, to thereby isolate a pure product or to significantly improve the purity to a level almost the same as that of the pure product (to obtain an isolated product).

The "arabinofuranosyl vitexin (specifically, 2"-O-α-D-arabinofuranosyl vitexin: see the following chemical formula 1)" and "vitexin (specifically, 8-β-D-glucopyranosyl-apigenin: see the following chemical formula 2)" " obtained as described above are kinds of C-glucoside flavonoids (flavonoid glycosides each having a C-glucoside skeleton).
In each of the compounds, an aglycone and a constituent sugar are bound via a C-glycoside bond, and hence it is difficult to decompose the compounds by heat, hydrolysis, an enzyme, or the like and the compounds are highly stable as flavonoid glycosides. Further, the compounds are glycosides and hence have water solubility, and in particular, the arabinofuranosyl vitexin has excellent water solubility.

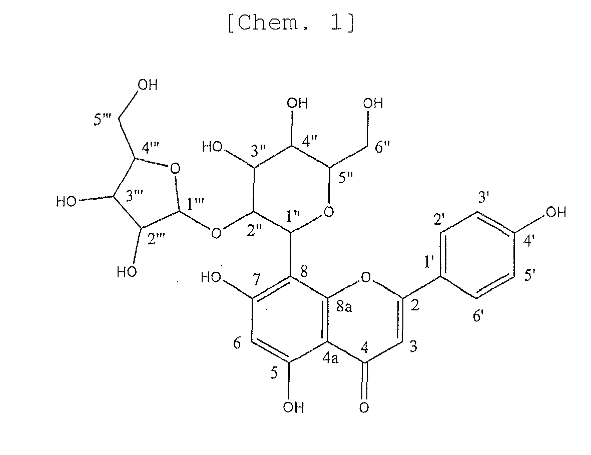

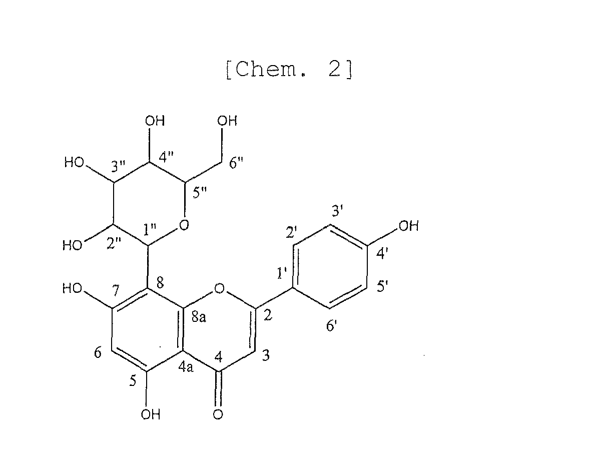

In the 'arabinofuranosyl vitexin, ' glucose and the arabinose moiety are bound via an O-glucoside bond, and hence the compound can be easily separated into the arabinose moiety and 'vitexin' (C-glucoside flavonoid main body) by hydrolysis with an enzyme or an acid.
It should be noted that, as described above, vitexin can be separated by performing a hydrolysis treatment during or after the extraction step, or after the purification step.

Further, the arabinofuranosyl vitexin and/or a hydrolysate thereof, i.e., vitexin (particularly, arabinofuranosyl vitexin) obtained as described above has a sedative activity and/or a sleep-inducing activity when orally ingested.
Therefore, an orally ingestible composition containing the above-mentioned compound has a sedative activity and/or a sleep-inducing activity.
It should be noted that the orally ingestible composition in the present invention has both the physiological functions, i.e., the sedative activity and the sleep-inducing activity, but in the case where the amount of the compound ingested as an active ingredient is small, only the sedative activity may be exhibited.

With regard to an effective dose of the orally ingestible composition in the present invention, when the arabinofuranosyl vitexin and/or a hydrolysate thereof, i.e., vitexin, used as an active ingredient, is orally ingested in an amount of 5 mg or more, preferably 20 mg or more per adult with a body weight of 60 kg per day, the sedative activity and/or the sleep-inducing activity can be obtained. Therefore, the composition may be ingested so as to ensure the required amount.

The content of 2"-O-α-D-arabinofuranosyl vitexin and/or a hydrolysate thereof, i.e., vitexin in the orally ingestible composition in the present invention may be an amount for securing the above-mentioned ingestion dose, and specifically, the content is 1 mass% or more, preferably 5 mass% or more.

The orally ingestible composition in the present invention may be formed into a food, a beverage, or a pharmaceutical preparation.
That is, the orally ingestible composition can be prepared as a food, a beverage, or a pharmaceutical preparation by mixing each of the compositions obtained by the above-mentioned steps ('composition directly containing the raw material,' 'solution extract,' or 'purified product') or an 'isolated product' with various raw materials.
It should be noted that the above-mentioned compound used as an active ingredient in the present invention is a C-glucoside flavonoid, and hence the compound has a stable bond between the constituent sugar and the flavonoidmoiety against heat or hydrolysis and has excellent water solubility. Therefore, the composition can be applied to a form to which an O-glucoside flavonoid or an aglycone thereof has had a difficulty in being applied.

For example, the composition may be formed into a food or beverage such as a biscuit, a snack, a chewing gum, a chewable tablet, a refreshing beverage, a drink, a soup, or a jelly.
Further, the composition may be formed into a pharmaceutical preparation such as a powder, ground particles, a granule, a capsule in which the composition is filled, a solution where the composition is dispersed in water, or a tablet obtained by blending the composition with a filler and the like.

### Examples

Hereinafter, the present invention is described by way of examples, but the scope of the present invention is not limited by the examples.

### [Example 1] Extraction of arabinofuranosyl vitexin from Basella alba

### (1) Extraction and purification of arabinofuranosyl vitexin

5 kg of aerial parts (mainly leaves) of raw *Basella alba* were cut into pieces, and 15 L of 80% ethanol were added to perform extraction at room temperature for 3 days, whereby obtaining an extract.
Next, the resultant extract was filtrated using a paper filter, and the filtrate was concentrated to dryness using a rotary evaporator. Then, 1 L of water was added thereto to extract water-soluble components, and centrifugation was carried out to obtain a supernatant.
The resultant supernatant was charged to DIAION HP20 (a porous synthetic adsorption resin column) preliminarily equilibrated with water, and unadsorbed components were removed with 3 L of water, followed by elution with 2 L of 20% ethanol.
After that, the resultant eluate was concentrated to dryness using a rotary evaporator, to thereby obtain 5.8 g of an arabinofuranosyl vitexin-purified product with a purity of about 85%. It should be noted that the purity of the arabinofuranosyl vitexin in the resultant purified product was measured by a preparative HPLC column (TSK GEL ODS, manufactured by Tosoh Corporation, 4.6 mm×25 cm) using a mobile phase of 25% (v/v) acetonitrile based on a peak area ratio.

### (2) Identification of arabinofuranosyl vitexin

200 mg of the arabinofuranosyl vitexin-purified product obtained as described above were charged to a TSK GEL ODS (manufactured by Tosoh Corporation, 20 mm×25 cm) preparative HPLC column, and elution was carried out using a mobile phase of 20% (v/v) acetonitrile. A fraction of a target peak was collected to isolate a pure product of the arabinofuranosyl vitexin.
Then, the resultant arabinofuranosyl vitexin-isolated product was subjected to MALDI-TOF mass spectrometry (manufactured by Applied Biosystems) to measure the molecular weight.
Further, structural analysis was performed by ¹H-NMR and ¹³C-NMR. JNM-AL400 (NMR) (manufactured by JEOL Ltd. ) was used as an apparatus for measurement and the measurement was performed by detecting signals in DMSO-d6 at 400 MHz for ¹H-NMR and at 100 MHz for ¹³C-NMR. δH (ppm) and δC (ppm) were measured for ¹H and ¹³C of the substance of interest, respectively. Table 1 shows the results.
It should be noted that, in Table 1, symbols on the right side of numerical values of δH (ppm) represent signal splitting patterns: "s" represents a singlet; "d" represent a doublet; "dd" represents a doublet of doublets; and "m" represents a multiplet.

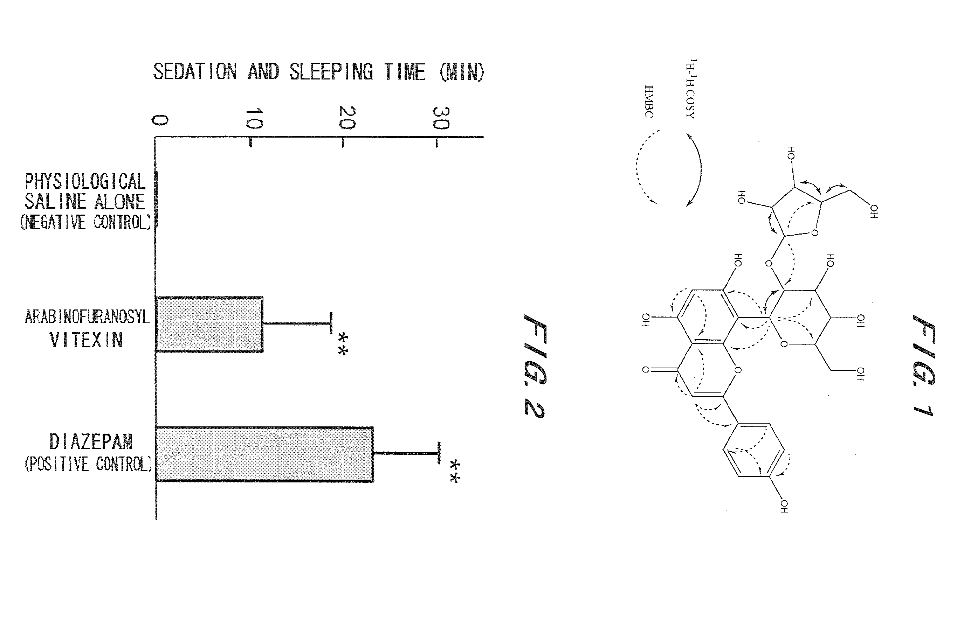

Then, the resultant product was subjected to COSY, DEPT, HMQC, and HMBC analyses.
The molecular structure formula, ¹H-¹HCOSY and HMBC signals, determined from the overall results of the analyses, are collectively shown in FIG. 1.

**[Table 1]**

| Carbon No. | δC ppm) | δH (ppm) | Carbon No. | δC (ppm) | δH (ppm) |
|---|---|---|---|---|---|
| 1 | | | 1 " | 71.8 | 4.75 d |
| 2 | 163.7 | | 2 " | 74.6 | 4.01 t |
| 3 | 102.2 | 6.74 s | 3 " | 79.1 | 3.43 m |
| 4 | 181.9 | | 4 " | 70.7 | 3.40 m |
| 4a | 103.7 | | 5" | 81.7 | 3.23 m |
| 5 | 160.3 | | 6" | 61.1 | 3.52 m, 3.75 m |
| 6 | 98.1 | 6.20 s | | | |
| 7 | 162.9 | | 1"' | 107 . 6 | 4.91 d |
| 8 | 104.2 | | 2"' | 82.0 | 3.60 dd |
| 8a | 156.1 | | 3"' | 75.7 | 3.55 m |
| | | | 4"' | 82.7 7 | 2.40 m |
| 1' | 121.7 | | 5"' | 58.9 | 2.89 m, 3.01 m |
| 2' | 128.9 | 8.03 d | | | |
| 3' | 116.0 | 6.89 d | | | |
| 4' | 161.1 | | | | |
| 5' | 116.0 | 6.89 d | | | |
| 6' | 128.9 | 8.03 d | | | |

The results of the MALDI-TOF mass spectrometry and NMR revealed that the arabinofuranosyl vitexin obtained in the foregoing is an arabinofuranosyl vitexin having a molecular weight of 564.51 (m/z 565. 51 [M+H]+) and a molecular formula of C₂₆H₂₈O₁₄.
Further, COSY and HMBC analyses were performed with respect to the results of measurement of δH (ppm) and δC (ppm) for ¹H and ¹³C of the substance of interest by NMR, and the results of the analyses suggested that, of whole carbon signals, signals at 107.6 ppm (No. 1"'), 82.0 ppm (NO. 2"'), 75.7 ppm (No. 3"'), 82.7 ppm (NO. 4"'), and 58. 9 ppm (No. 5"') were attributed to carbon atoms constructing arabinose.
Further, similar analysis suggested that signals at 71.8 ppm (No. 1"), 74.6 ppm (No. 2"), 79.1 ppm (No. 3"), 70.7 ppm (No. 4"), 81.7 ppm (No. 5"), and 61.1 ppm (No. 6") were attributed to carbon atoms constructing glucose.
It should be noted that the other carbon and hydrogen signals were analyzed in similar ways and were found to be attributed to carbon and hydrogen atoms constructing vitexin.

In addition, of the above-mentioned five carbon atoms constructing arabinose, the signal at 107 . 6 ppm (No. 1"') observed in a high magnetic field was considered to be an anomeric carbon relating to a glucose bond.
From a methyl proton (4.91 ppm) corresponding to the signal, an HMBC signal was observed in the carbon atom at 74.6 ppm (NO. 2 ") that constructs glucose.
This suggested that "arabinoseposition 1"' is directlybonded to glucose position 2 " with a glucoside bond."

The results in the foregoing confirm that the compound obtained by isolation from the *Basella alba* extract by the above-mentioned method is 2"-O-α-D-arabinofuranosyl vitexin (C-glucoside flavonoid shown in Fig. 1.

### [Example 2] Sedative and sleep-inducing activities of arabinofuranosyl vitexin

### (1) Purification of arabinofuranosyl vitexin

1.5 g of the arabinofuranosyl vitexin-purified product with a purity of about 85% obtained in Examples 1(1) was subjected to flash chromatography (Hi FLASH column, 26×100 mm, silica gel 40 µm, 30 g, manufactured by YAMAZEN CORPORATION) using a solvent system of chloroform/methanol/water (65:25:4), and a target fraction was collected.
The resultant eluate was concentrated to dryness using a evaporator and under a stream of nitrogen gas, to thereby obtain 720 mg of an arabinofuranosyl vitexin-isolated product (showing a single spot on TLC) .

### (2) Study on sedative and sleep-inducing activities

The arabinofuranosyl vitexin-isolated product obtained in the above-mentioned procedure (1) was used to study sedative and sleep-inducing effects. A sample was orally administered to six ICR mice (body weight: 40 to 45 g).
As the sample, the 'arabinofuranosyl vitexin (100 mg/kg body weight) ' dissolved in physiological saline was orally administered. It should be noted that 'diazepam (2 mg/kg body weight)' having a sleep-inducing effect was dissolved in physiological saline and orally administered as a positive control,and'physiological saline alone' was orally administered as a negative control.
Then, 45 minutes after the oral administration of the sample, thiopental sodium (30 mg/kg body weight) capable of activating behavior of a mouse was administered intraperitoneally, and the mice were individually placed in mouse cages (17 cm×27 cm).
In a period from the start of the administration of thiopental sodium to 40 minutes later, a total time when a mouse stopped moving for one or more minutes and got in a sleep state was measured to evaluate the sedative and sleep-inducing effects.
It should be noted that the symbol' '**' in FIG. 2 represents that there is a significant difference compared with the negative control group at a risk rate of less than 1% (p<0.01) by a Dunnet multiple comparison assay.

As shown in FIG. 2, it was revealed that the sedation and sleeping times were extended significantly compared with the negative control group (p<0.01), and the arabinofuranosyl vitexin (2"-O-α-D-arabinofuranosyl vitexin) had sedative and sleep-inducing activities.

### [Example 3] Preparation of vitexin by hydrolysis using acid

100 ml of 0.4N HCl were added to 1 g of the arabinofuranosyl vitexin-purified product with a purity of about 85% obtained in Example 1(1), and the mixture was heated at 100°C for 20 minutes and centrifuged at 3,000 rpm for 15 minutes, to thereby obtain insoluble matter.
The insoluble matter was suspended in 20 ml of water, and the mixture was centrifuged at 3,000 rpm for 15 minutes, to thereby obtain insoluble matter again. The procedure was repeated three times.
Next, 10 ml of ethanol were added to the resultant insoluble matter, and the mixture was stirred and centrifuged in the same manner as described above, to thereby obtain insoluble matter. The procedure was repeated three times. Then, ethanol remaining in the resultant insoluble matter was dried, to thereby obtain 480 mg of a pure product of 'vitexin.'
It should be noted that the fact that the resultant substance was vitexin was confirmed by the fact that the retention time and UV spectrum correspond to those of a standard substance of vitexin (8-β-D-glucopyranosyl-apigenin) (manufactured by ChromaDex, Inc.) in an HPLC analysis.

### Industrial Applicability

In the present invention, the arabinofuranosyl vitexin and/or a hydrolysate thereof, i. e. , vitexin, extracted from *Basella alba* used as a raw material, has excellent physiological functions, in particular, sedative and sleep-inducing activities. Therefore, the compounds can be used as a safe component from a naturally occurring edible plant in food, beverage, and pharmaceutical preparation fields in high demand in modern society having social issues such as anxiety, insomnia, and sleep disorders.

## Claims

1. A production method for an orally ingestible composition containing an arabinofuranosyl vitexin and/or a hydrolysate thereof, i.e., vitexin, the method comprising using *Basella alba* as a raw material.

2. A production method for an orally ingestible composition according to claim 1, wherein the arabinofuranosyl vitexin is 2"-O-α-D-arabinofuranosyl vitexin.

3. A production method for an orally ingestible composition according to claim 1 or 2, wherein the production method for an orally ingestible composition comprises the steps of: treating *Basella alba* used as the raw material by any one of cutting, fragmentating, and grinding; and performing solution extraction to prepare an extract.

4. A production method for an orally ingestible composition according to any one of claims 1 to 3, wherein the production method for an orally ingestible composition comprises the step of performing solution extraction with ethanol or ethanol having a final concentration of 55% or more to prepare an extract.

5. A production method for an orally ingestible composition according to any one of claims 1 to 4, wherein the production method for an orally ingestible composition comprises the step of purifying the resultant extract using a column after obtaining the extract.

6. A production method for an orally ingestible composition according to any one of claims 1 to 5, wherein the orally ingestible composition contains 2"-O-α-D-arabinofuranosyl vitexin and/or a hydrolysate thereof, i.e. , vitexin as an active ingredient and has a sedative activity and/or a sleep-inducing activity.

7. A food or beverage, comprising an orally ingestible composition obtained by the production method according to any one of claims 1 to 6.

8. A drug, comprising an orally ingestible composition obtained by the production method according to claim 6.

9. A drug, containing 2"-O-α-D-arabinofuranosyl vitexin as an active ingredient and having a sedative activity and/or a sleep-inducing activity.
